# EUROPEAN PATENT APPLICATION

(11) **EP 0 425 049 A2**
(43) Date of publication of application: **02.05.1991**
(21) Application number: 90202827.3
(22) Date of filing: 23.10.1990
(51) Int. Cl.: C08G 75/28

(54) **Polythiocarbonates and a process for their preparation**

(30) Priority: 26.10.1989 IT 2214589
(71) Applicant: ENICHEM S.p.A., I-20124 Milano (IT)
(72) Inventor: Berti, Corrado, I-48022 Lugo,Ravenna (IT); Della Fortuna, Giorgio, I-20151 Milan (IT); Manaresi, Piero, I-40139 Bologna (IT); Marianucci, Elisabetta, I-40127 Bologna (IT); Pilati, Francesco, I-40127 Bologna (IT); Poloni, Marino, I-40141 Bologna (IT)
(74) Representative: Fusina, Gerolamo

(57) **Abstract**

The aromatic and/or aromatic-aliphatic polythiocarbonates of the present invention are definable by the general formula (I):
-[-(-CO-O-R-O-)ₙ-CO-S-R′-S-]ₘ- (I)
where n, m, R and R′ have the meaning given in the text.

The polythiocarbonates corresponding to general formula (I) in which n=0 are prepared by reacting dithiophenols of formula HS-R′-SH with phosgene. The polythiocarbonates corresponding to general formula (I) in which n≠0 are prepared by reacting a polycarbonate prepolymer with dithiophenols of formula HS-R′-SH.

They are used in the production of articles with flame-retardant properties.

## Description

This invention relates to aromatic and/or aromatic-aliphatic polythiocarbonates, the processes for their preparation, and their use either as such or in polymer compositions in the production of articles with flame-retardant properties.

Several methods are known from the literature for making polycarbonates self-extinguishing. However many of these have proved unsatisfactory in that they negatively affect the other properties of the polymer.

For example the self-extinguishing property of polycarbonates can be improved by using relatively large quantities of additives in the form of halogenated organic compounds with or without the synergic effect of antimony oxide (J.T. Howarth et al. Plastic World pp 64-74 March 1973). It has however been found that these additives lead to excessive degradation of the polycarbonates during their working (US 3,334,154), with consequent deterioration in the main physical properties of the polymer (US 4,153,595).

It has also been proposed to use tetrahalogenated derivatives of bisphenol-A as co-monomers in copolymerization with bisphenol-A to give a polycarbonate which either alone or mixed with a non-halogenated polycarbonate represents material having increased flame resistance.

In this case it has also been reported that to attain good flame resistance such polymer materials must contain very large quantities of halogen (US 4,046,836, US 3,775,367, US 4,100,130), which results in:
i) a marked decay in behaviour during working, so that the mechanical characteristics do not show the typical level of non-halogenated polycarbonate;
ii) the development of hydrohalogen acid during normal transformation, causing machinery corrosion;
111) during combustion, the development of large quantities of halogenated degradation compounds, and in particular hydrohalogen acids which can cause further damage to metal structures by corrosion.

More recent discoveries are also known by which polycarbonate self-extinguishing is improved, involving the addition of small quantities of particular organic compounds such as alkaline or alkaline-earth salts of aryl or alkylsulphonic acids or their derivatives (for example US 3,940,366, US 3,933,734, US 4,104,253, US 4,153,195, US 4,214,062).

The identification of flame-retardant activity in these particular compounds was the result of trials conducted on different classes of organic compounds, which demonstrated amongst other things the inadequacy of using carboxylic acid salts as flame retardants because of the undesirable side effects induced especially on the stability of the resin at transformation temperatures (V. Mark, Organic Coatings and Plastics Chemistry, vol. 43, Preprints of papers presented at the 2nd Chem. Congress of the North American Continent, 1980, August 24-29, S.Francisco page 71).

The object of the present invention is therefore to provide a new class of sulphur-containing polycarbonates characterised by flame-retardant behaviour.

In particular, according to the present invention a new class of polycarbonates has been discovered the molecules of which contain sulphur atoms, giving them good flame-retardant properties. These polycarbonates can be prepared in a simple and convenient manner and are also characterised by good thermal stability under working conditions. In accordance therewith, a first aspect of the present invention relates to new aromatic or mixed aromatic-aliphatic polythiocarbonates definable by the general formula (I):
-[-(-CO-O-R-O-)ₙ-CO-S-R′-S-]ₘ- (I)
where n is a number between 0 and 20 and m is a number between 5 and 50;
R represents a bivalent C₂-C₁₀ linear or branched chain alkyl radical, a bifunctional aryl group or groups of the type: 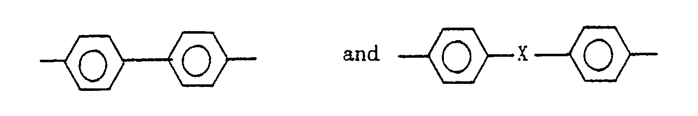 where X represents a -CH₂-, 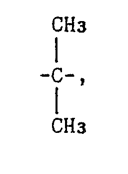 SO₂, -S- or -O- group
and R′ represents a bifunctional aryl group which can also substituents in the benzene ring, or groups of the type: 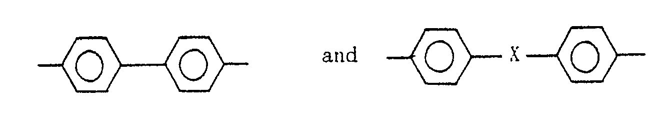 where X has the aforesaid meaning.

In particular, R and R′, which can be identical or different, can also represent a group chosen from 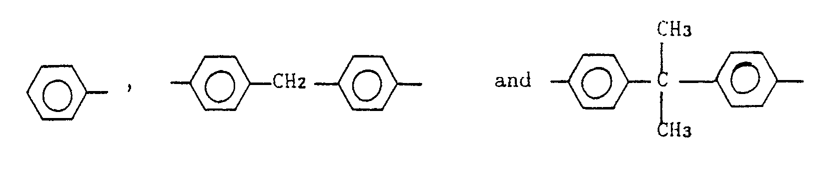 in which the benzene rings can be simple or substituted.

A further preferred aspect of the present invention is an aromatic polythiocarbonate corresponding to the general formula (II)
-(-CO-S-R′-S-)-ₘ (II)
where m and R′ have the aforesaid meaning, and in particular within the general formula (II) the polythiocarbonate (III): 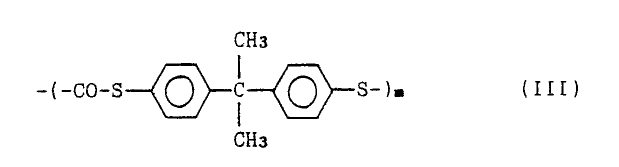 where m has the aforesaid meaning.

For the purposes of the present invention the term "aryl" indicates any C₆-C₁₄ aromatic group, which may be substituted. By way of example, substituents which can be present in the ring include alkyl, haloalkyl or halogen groups.

In accordance with general formula (1), the polythiocarbonates of the present invention have a sulphur content which can be adjusted as required, and is maximum for n=0.

Thus if n=0 and R′ is represented by the bifunctional group 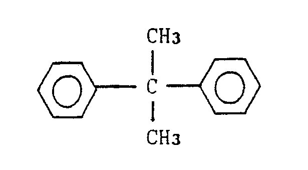 ie the case of the polythiocarbonate (III), the sulphur content is 22.4% by weight. This maximum value reduces as the value of n increases.

The polycarbonates describable by the general formula (I) are prepared by reacting a polycarbonate prepolymer with dithiophenols of formula HS-R′-SH. The reaction is conducted in the presence of a two-phase system. When n is zero the polythiocarbonates of general formula (I) assume the structure corresponding to general formula (II) and can be prepared by reacting aromatic dithiols HS-R′-SH with phosgene in the presence of an alkaline base, by synthesis methods similar to those used for preparing the corresponding polycarbonates starting from diphenols.

The preferred reaction conditions are those in which the process is conducted at a temperature of 20-25°C using a halogenated hydrocarbon as the polymer solvent and a tertiary aliphatic amine or a tetraalkylammonium salt as catalyst.

Two or more aromatic dithiols mixed together can be used in the synthesis.

The various polymers of the present invention can be used either alone or mixed with other polymers or in polymer blends, for producing articles having self-extinguishing properties.

The polymers of the present invention can also contain other additives such as pigments, thermooxidative stabilizers, lubricants, dyes, fillers, U.V. radiation absorbents etc.

The following examples are provided to further illustrate the invention but without limiting its scope.

### EXAMPLE 1

### Synthesis of the polythiocarbonate (III)

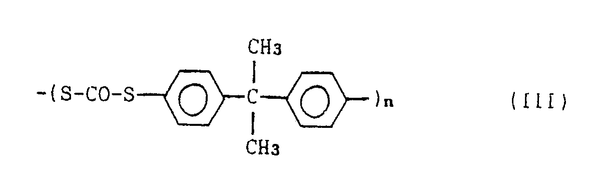

0.4 g (1.54 mmoles) of 2,2-bis(4-mercaptophenyl)propane (BTA), 0.05 g (0.22 mmoles) of Et₃PhCH₂NCl as catalyst, 0.28 g (6.93 mmoles) of NaOH, 10 ml of water and 10 ml of CH₂Cl₂ were fed into a reaction flask under a stream of nitrogen while maintaining vigorous stirring. 0.23 g (2.3 mmoles) of phosgene dissolved in 10 ml of CH₂Cl₂ were added dropwise to the mixture contained in the flask while maintaining vigorous stirring.

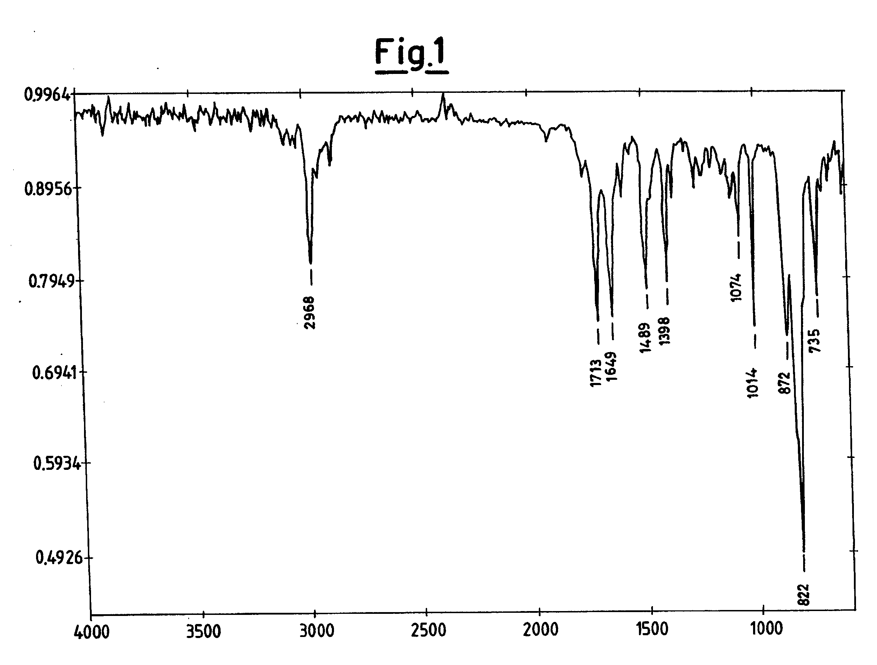

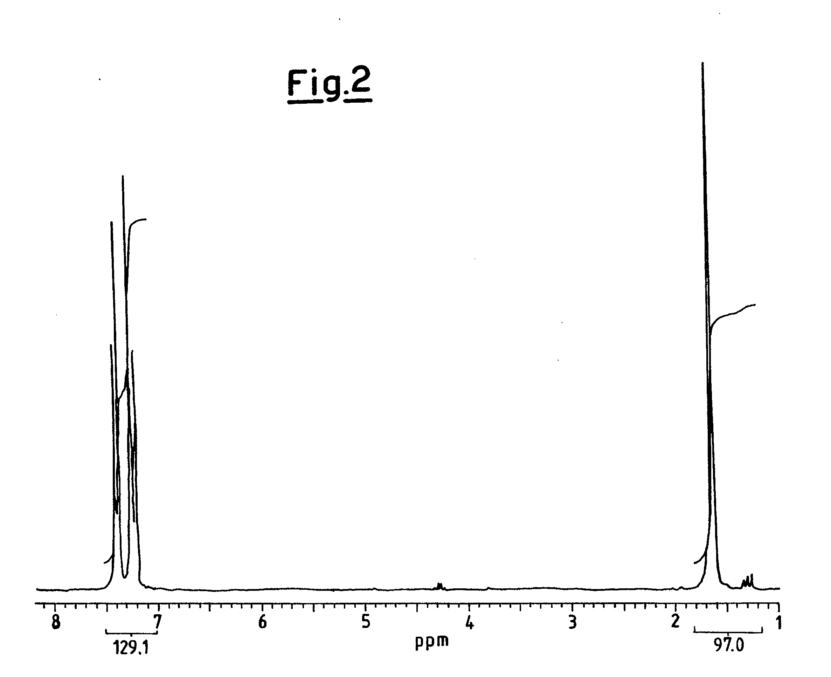

After 1 hour of reaction at a temperature of 25°C the organic phase was separated, washed with 10 wt% HCl (10 ml) and water (twice with 20 ml portions) and then added slowly to 200 ml of methanol. The precipitated polymer was filtered and dried under vacuum at 70°C overnight.
0.31g of white polymer were obtained (yield 70%) having an intrinsic viscosity (in CHCl₃ at 30°C) of 0.14 dl/g.
I.R. and ¹H-NMR (in CDCl₃) spectra confirm the polymer structure (Figures 1 and 2).

### EXAMPLE 2

### Synthesis of the copolymer of formula

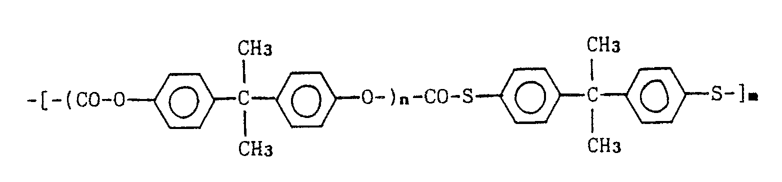

0.35 g (1.35 mmoles, 2.7 meq (SH) of BTA, 0.3 g (7.5 mmoles) of NaOH and 0.05 g (0.22 mmoles) of Et₃PhCH₂NCl as catalyst were dissolved under nitrogen in 10 ml of water and 10 ml of CH₂Cl₂ were added to the solution. A solution formed from 5 ml of polycarbonate prepolymer solution (1.42 g of dry matter, 3.4 meq of chloroformate terminals and 0.7 meq of phenolic terminals) and 5 ml of CH₂Cl₂ was added dropwise to the mixture while maintaining vigorous stirring.

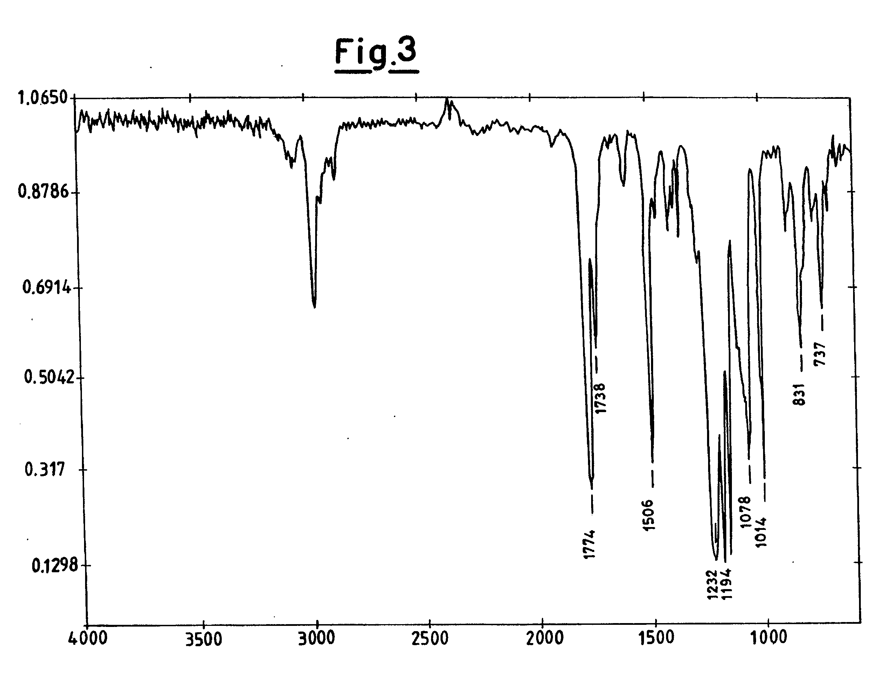

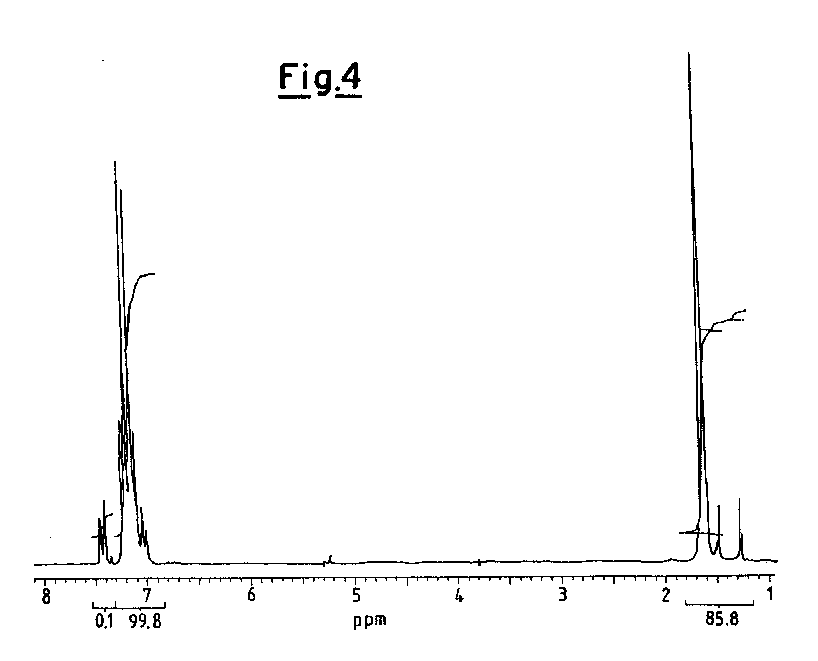

After 1 hour of reaction the organic phase was separated, washed with 10 wt% HCl (10 ml) and water (twice with 20 ml portions) and then added slowly to 200 ml of methanol. The precipitated polymer was filtered and dried under vacuum at 70°C overnight.
1.7 g of white polymer were obtained (yield 96%) having an intrinsic viscosity (in CHCl₃ at 30°C) of 0.44 dl/g.
I.R. and ¹H-NMR (in CDCl₃) spectra confirm the polymer structure (Figures 3 and 4).

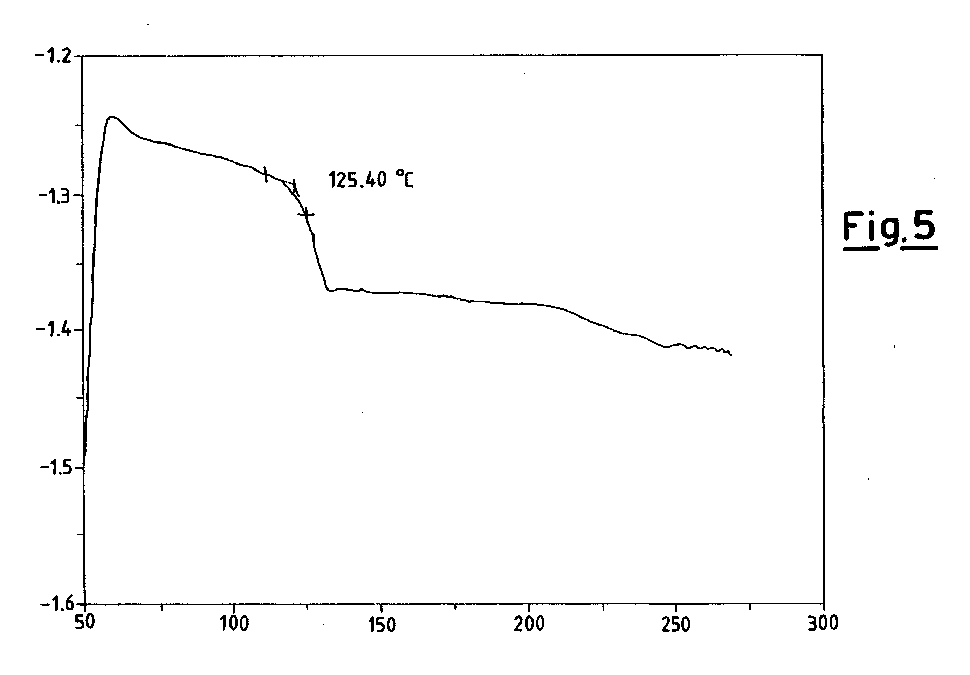

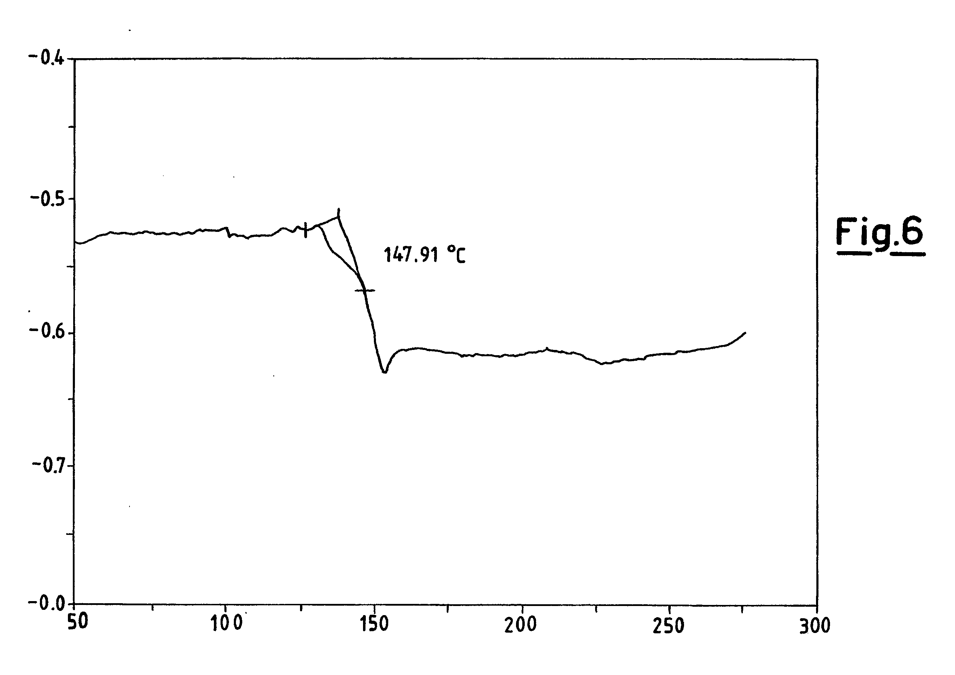

The polymers of Examples 1 and 2 were analyzed with DSC (Figures 5 and 6). In neither case was fusion observed. The Tg values were 125°C for the polythiocarbonate (III) and 148°C for the copolymer of Example 2.

The said figures must be read in the following manner:
- Figure 1 shows the IR spectrum for the polythiocarbonate prepared in Example 1. The horizontal axis represents wave numbers (in cm⁻¹) and the vertical axis represents transmittance;
- Figure 2 shows the ¹H-NMR spectrum for the same polythiocarbonate;
- Figure 3 shows the IR spectrum for the copolymer of Example 2, with the same axes as in Figure 1;
- Figure 4 shows the ¹H-NMR spectrum for the copolymer of Example 2;
- Figure 5 shows the DSC curve for the polythiocarbonate of Example 1, the horizontal axis representing temperature and the vertical axis representing heat flow (W/g);
- Figure 6 shows the same for the copolymer of Example 2.

## Claims

1. Aromatic and/or aromatic-aliphatic polythiocarbonates definable by the general formula (I):
-[-(-CO-O-R-O-)ₙ-CO-S-R′-S-]ₘ- (I)
where n is a number between 0 and 20 and m is a number between 5 and 50;
R represents a bifunctional C₂-C₁₀ linear or branched chain alkyl radical, a bifunctional aryl group or a bifunctional group of the type: 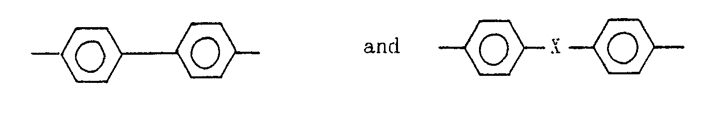 where X represents a -CH₂-, 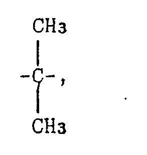 SO₂, -S- or -O- group
and R′ represents a bifunctional aryl group which can also have substituents in the benzene ring, or a bifunctional group of the type: 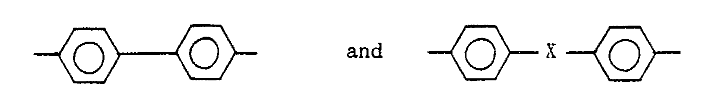 where X has the aforesaid meaning.

2. A polythiocarbonate as claimed in claim 1, wherein R and R′, which can be identical or different, represent a group of the type 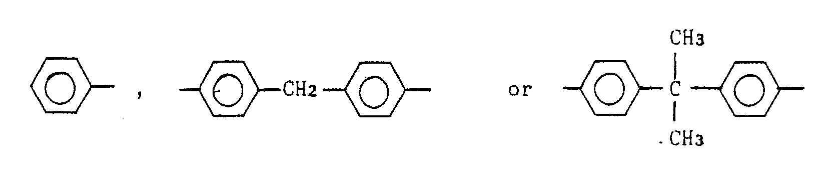 in which the benzene rings can be simple or substituted.

3. A polythiocarbonate as claimed in claim 1, characterised by the corresponding general formula (II)
-(-CO-S-R′-S-)-ₘ (II)
where m and R′ have the aforesaid meaning.

4. A polythiocarbonate as claimed in claim 2, characterised by the corresponding general formula (III): 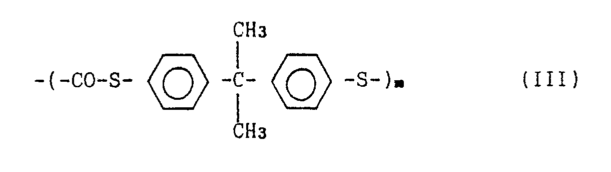 where m has the aforesaid meaning.

5. A process for preparing the polythiocarbonates of general formula (I), characterised by reacting a polycarbonate with an aromatic dithiol in the presence of a two-phase system.

6. A process as claimed in claim 5, characterised in that the two-phase system is an H₂O/CH₂Cl₂ mixture.

7. A process as claimed in claims 6 and 7, characterised in that the reaction takes place at a temperature of between 20 and 40°C for a time of between 10 minutes and 3 hours.

8. A process for preparing the polythiocarbonates of general formula (II), consisting of reacting an aromatic dithiol with phosgene.

9. A process as claimed in claim 8, characterised in that the reaction takes place at a temperature of between 20 and 40°C.

10. A process for preparing polythiocarbonates as claimed in claim 8, characterised in that the reaction is effected for a time of between 30 minutes and 2 hours.

11. A process for preparing polythiocarbonates as claimed in claim 8, characterised in that the reaction is conducted in a two- phase system.

12. A process for preparing polythiocarbonates as claimed in claim 11, characterised in that the two-phase system consists of water and methylene chloride.
